# EUROPÄISCHE PATENTSCHRIFT

(11) **EP 3 602 012 B1**
(45) Veröffentlichungstag und Bekanntmachung des Hinweises auf die Patenterteilung: **15.02.2023**
(21) Anmeldenummer: 18716958.6
(22) Anmeldetag: 29.03.2018
(51) Int. Cl.: G01N 21/33, G01N 21/61, G01N 21/3504, G01N 33/00

(54) **GASANALYSATOR**
GAS ANALYSER
ANALYSEUR DE GAZ

(30) Priorität: 31.03.2017 DE 202017001743 U
(43) Veröffentlichungstag der Anmeldung: 05.02.2020
(73) Patentinhaber: Siemens Aktiengesellschaft, 80333 München (DE)
(72) Erfinder: HEFFELS, Camiel, 76297 Stutensee-Büchig (DE)
(86) Internationale Anmeldenummer: PCT/EP2018/058230
(87) Internationale Veröffentlichungsnummer: WO 2018/178306

(56) Entgegenhaltungen:
- WO-A1-2014/157282
- WO-A1-2014/162537
- WO-A1-2016/145066
- DE-A1-102014 107 342
- US-A1- 2016 054 294
- US-B1- 6 791 689
- US-B2- 9 000 374
- GOMEZ A L ET AL: "Fast response cavity enhanced ozone monitor", ATMOSPHERIC MEASUREMENT TECHNIQUES, Bd. 6, Nr. 2, 2013, Seiten 487-494, XP055476579, DOI: 10.5194/amt-6-487-2013
- Thorlabs: "30 mm Cage Components", , 2016, XP055477282, Gefunden im Internet: URL:https://web.archive.org/web/2016040121 0617/https://www.thorlabs.de/navigation.cf m?guide_id=2004 [gefunden am 2018-05-22]
- Anonymous: "GCH25-75 Gas Cell Oven", , 19. Mai 2010 (2010-05-19), XP055411168, Gefunden im Internet: URL:https://www.thorlabs.com/drawings/1d91 e8af276bf034-D5CF10CC-92C2-AA6A-6E14871368 ECB5E2/GCH25-75-Manual.pdf [gefunden am 2017-09-28]

## Beschreibung

Die Erfindung betrifft Gasanalysator zur Messung von zwei Gaskomponenten in einem Messgas nach dem Oberbegriff von Anspruch 1.

Ein derartiger Gasanalysator ist aus der WO 2014/162537 A1 bekannt.

Aus Ryoichi Higashi et al.: "A NOx and SO2 gas analyzer using deep-UV and violet light-emitting diodes for continuous emissions monitoring systems", Proc. SPIE 9003, Light-Emitting Diodes: Materials, Devices, and Applications for Solid State Lighting XVIII, 90031F (February 27, 2014), ist ein Gasanalysator zur Messung von Stickoxiden und Schwefeldioxid in einem Abgas bekannt, wobei in dem Abgas enthaltenes Stickstoffmonoxid in mit dem Gasanalysator messbares Stickstoffdioxid umgesetzt wird, so dass die von dem Gasanalysator ermittelte Konzentration von Stickstoffdioxid ein Maß für die Konzentration von Stickoxiden in dem Abgas ist.

Bei dem bekannten Gasanalysator sind eine erste Leuchtdiode mit einer Emissionswellenlänge von etwa 280 nm im Absorptionsbereich von Schwefeldioxid und eine zweite Leuchtdiode mit einer Emissionswellenlänge von etwa 400 nm im Absorptionsbereich von Stickstoffdioxid dicht nebeneinander in einem LED-Array angeordnet. Ihr Licht wird mittels einer Kollimatorlinse zu einem parallelen Lichtbündel geformt, das eine Messkammer durchstrahlt und anschließend auf einen Messdetektor fokussiert wird. Mittels eines Strahlteilers zwischen der Kollimatorlinse und der Messkammer wird ein Teil des Lichts auf einen Referenzdetektor gelenkt.

Aus Gomez, A. L. et al.: "Fast response cavity enhanced ozone monitor", Atmos. Meas. Tech., 6, 487-494, ist ein Gasanalysator bekannt, bei dem das Licht einer Leuchtdiode mithilfe von zwei Strahlteilern in zwei Lichtstrahlen durch zwei Messkammern aufgeteilt und hinter diesen von zwei Messdetektoren detektiert wird. Die Leuchtdiode, Strahlteiler und Messdetektoren sind in Metallblöcken gehalten, die auf Käfigstangen eines Thorlabs 30 mm Cagemount Systems aufgeschoben und an diesen mithilfe von Fixierschrauben fixiert sind.

Die US 6,791,689 B1 offenbart einen Gasanalysator, bei dem das Licht von zwei Leuchtdioden mithilfe eines Strahlteilers durch eine Messkammer hindurch auf einen Messdetektor geleitet wird.

Die WO 2014/157282 A1 offenbart einen Gasanalysator, bei dem das Licht von zwei Leuchtdioden mithilfe eines Strahlteilers unmittelbar auf einen Referenzdetektor und durch eine Messkammer hindurch auf einen Messdetektor geleitet wird.

Die US 9,000,374 B2 offenbart einen Gasanalysator, bei dem das Licht von zwei Leuchtdioden mithilfe eines Strahlteilers in ein optisches Kabel eingeleitet wird. Der Strahlteiler ist in einem quaderförmigen Metallblock in dem Kreuzungsbereich von zwei Bohrungen angeordnet. Die Leuchtdioden sind in Metallblöcken gehalten, die auf Käfigstangen aufgeschoben und an diesen mithilfe von Fixierschrauben fixiert sind.

Die US 2016/054294 A offenbart einen Gasanalysator, bei dem die Messkammer mit teiltransparenten Hohlspiegeln abgeschlossen ist, die zwischen sich einen Resonator zur Verlängerung der Absorptionsstrecke bilden.

Die WO 2016/145066 A1 offenbart ein Photometer zur Messung der Konzentration von einem Chromophor oder mehreren Chromophoren in einem Fluid.

Der Erfindung liegt die Aufgabe zugrunde, einen Gasanalysator zur Messung von zwei Gaskomponenten in einem Messgas anzugeben, der sich durch einen einfachen und stabilen Aufbau auszeichnet.

Gemäß der Erfindung wird die Aufgabe durch den in Anspruch 1 angegebenen Gasanalysator gelöst, von dem vorteilhafte Weiterbildungen in den Unteransprüchen angegeben sind.

Der erfindungsgemäße Gasanalysator weist einen kompakten Aufbau auf. Die Messkammer, der den Strahlteiler enthaltende Metallblock und der Detektorblock sind aufgrund der Verspannung über die Zugstangen in einem engen thermischen Kontakt, so dass auch die daran angeordneten Leuchtdioden und Detektoren auf gleichem Temperaturniveau sind.

Die Zugstäbe weisen an ihren Enden Gewinde auf oder sind als Gewindestäbe ausgebildet. Sie können unmittelbar mit dem quaderförmigen Metallblock und dem Detektorblock verbunden sein oder, wie weiter unten noch erläutert wird, mittelbar über außen an dem Metallblock und Detektorblock anliegende Platten auf diese wirken.

Der die Messkammer bildende Hohlkörper kann als Hohlprofilkörper ausgebildet sein oder eine durchgehende Bohrung enthalten, die in einen Langkörper aus Metall eingefräst ist.

Zur Vereinfachung der Montage und Justierung des Strahlteilers enthält der quaderförmige Metallblock auf seiner Deckfläche vorzugsweise eine in den Kreuzungsbereich der beiden Bohrungen hineinreichende Öffnung, in der ein den Strahlteiler aufnehmender Einsatz eingesetzt ist.

Die Leuchtdioden, der Messdetektor und/oder der Referenzdetektor sind vorzugsweise auf Platten angeordnet, welche entweder unmittelbar oder gegebenenfalls unter Zwischenlage eines Abstandsrahmens an den jeweiligen Seitenflächen des quaderförmigen Metallblocks oder im Falle des Referenzdetektors an der von der Messkammer abgewandten Seite des Detektorblocks montiert sind. Die Platten können jeweils für sich an dem Metallblock bzw. dem Detektorbock montiert, z. B. mit diesem verschraubt sein. Im Falle des Messdetektors und der ihm auf gerader Linie gegenüberliegenden Leuchtdiode können die oben erwähnten Zugstangen durch Löcher oder Bohrungen in dem Detektorblock, dem Metallblock und den den Messdetektor und die Leuchtdiode tragenden Platten hindurch laufen und an den nach außen gewandten Seiten der Platten mit diesen verschraubt sein, so dass der Detektorblock, die Messkammer und der Metallblock zwischen den Platten eingespannt sind.

Das Licht der Leuchtdioden wird vorzugsweise mit Hilfe von Kollimatorlinsen zu einem parallelen Lichtbündel geformt, das mit Hilfe von Fokussierlinsen auf den Referenzdetektor bzw. nach Durchstrahlen der Messkammer auf den Messdetektor fokussiert wird. Der damit verbundene konstruktive Aufwand lässt sich besonders gering halten, wenn die Kollimatorlinsen mit ihren Linsenhalterungen in den Bohrungen auf den den Leuchtdioden zugewandten Seitenflächen des Metallblocks eingesetzt sind und die Fokussierlinsen ihren Linsenhalterungen in der Bohrung auf der dem Referenzdetektor gegenüberliegenden Seitenfläche des Metallblocks und in der Öffnung des Detektorblocks gegenüber dem Messdetektor eingesetzt ist. Vorzugsweise haben alle eingesetzten Linsen dieselbe technische Spezifikation.

Durch Erwärmen der Messkammer, beispielsweise auf 52 °C, kann verhindert werden, dass in dem Messgas enthaltener Wasserdampf auskondensiert. Zu diesem Zweck ist vorzugsweise an der Außenwand des die Messkammer bildenden Hohlkörpers mindestens ein elektrisches Heizelement montiert. Hierzu eignet sich insbesondere ein Transistor, der gleichzeitig als Heizelement und Temperaturfühler dient, so dass keine aufwendige Regelung zur Konstanthaltung der Temperatur erforderlich ist. Aufgrund des guten thermischen Kontakts werden die Leuchtdioden und Detektoren über den quaderförmigen Metallblock und den metallenen Detektorblock mit temperiert, so dass Messfehler aufgrund sich ändernder Temperaturen vermieden werden können. Zur Konstanthaltung der Temperatur dient auch die bevorzugte Unterbringung des Gasanalysators in einem aus einem schalenförmigen Unterteil und einem schalen- oder deckelförmigen Oberteil bestehenden Behälter aus Dämmstoff, beispielsweise Polystyrol-Hartschaum oder expandiertem Polypropylen (EPP).

Die Messkammer kann in bekannter Weise an beiden Enden mit planen Fenstern oder alternativ mit teiltransparenten Hohlspiegeln abgeschlossen sein, die zwischen sich einen Resonator zur Verlängerung der Absorptionsstrecke bilden. Das entsprechende Messverfahren "Incoherent Broad-Band Cavity-Enhanced Absorption Spectroscopy" (IBBCEAS) vereint die Einfachheit und Zuverlässigkeit der klassischen Absorptionsspektroskopie mit der Empfindlichkeit der "Cavity-Ring-Down" Spektroskopie.

Im Weiteren wird die Erfindung anhand von Ausführungsbeispielen und unter Bezugnahme auf die Figuren der Zeichnung erläutert; im Einzelnen zeigen
- Figur 1: ein stark vereinfachtes Blockschaltbild und
- Figur 2: eine perspektivische Explosionsansicht des erfindungsgemäßen Gasanalysators.

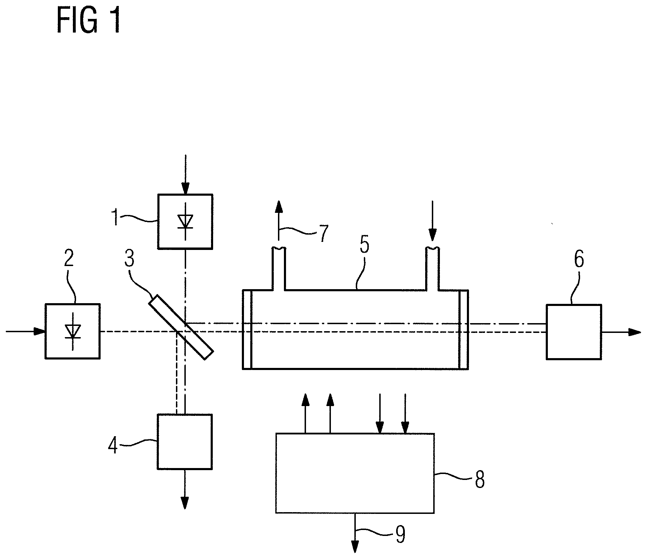

Figur 1 zeigt einen Gasanalysator, der eine erste Leuchtdiode 1 mit einer Emissionswellenläge von 285 nm (+/- 10nm)im Absorptionsbereich von Schwefeldioxid und eine zweite Leuchtdiode 2 mit einer Emissionswellenläge von 400 nm (+/- 20nm) im Absorptionsbereich von Stickstoffdioxid enthält. Das Licht beider Leuchtdioden 1, 2 wird über einen Strahlteiler 3 unmittelbar auf einen Referenzdetektor 4 und durch eine Messkammer 5 hindurch auf einen Messdetektor 6 geleitet. Die Messkammer 5 wird von einem Messgas 7 durchströmt, dessen Schwefeldioxid- und Stickstoffdioxid-Gehalt gemessen werden soll. Eine Steuer- und Auswerteeinrichtung 8 steuert die Leuchtdioden 1, 2 abwechselnd an und wertet die von dem Messdetektor 6 und dem Referenzdetektor 4 gelieferten Signale zu einem Messergebnis 9 für den Schwefeldioxid- und Stickstoffdioxid-Gehalt des Messgases 7 aus. Das von dem Messdetektor 6 gelieferte Signal ist von der wellenlängenspezifischen Absorption durch die beiden interessierenden Gaskomponenten und damit deren Konzentration abhängig und wird mit dem Signal des Referenzdetektors normiert.

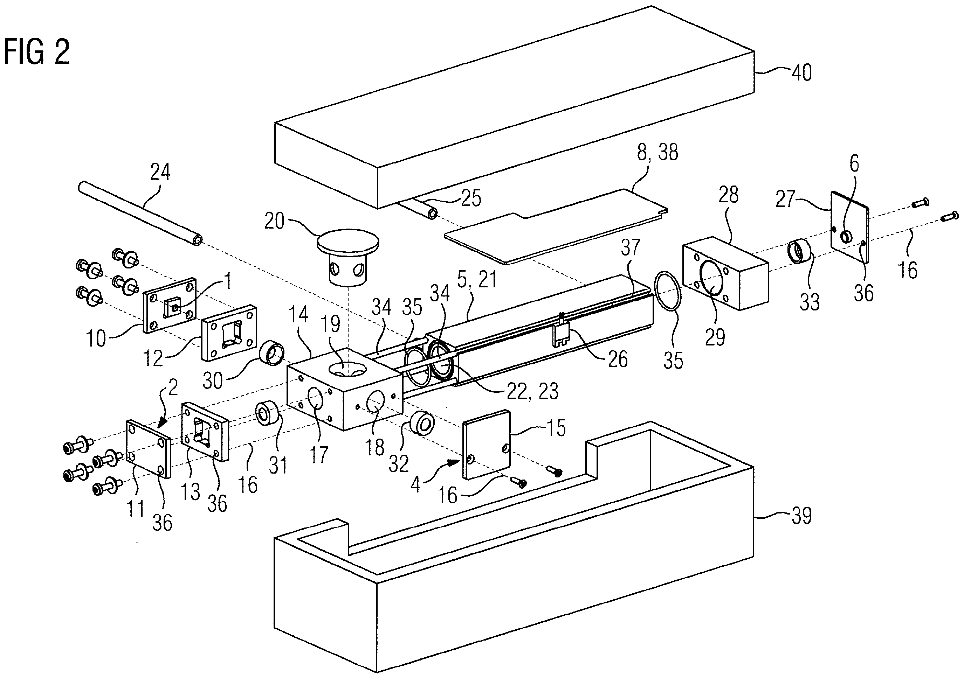

Figur 2 zeigt die auf einer Platte 10 montierte Leuchtdiode 1 und eine baugleiche Platte 11, auf der die hier verdeckte Leuchtdiode 2 moniert ist. Beide Platten 10, 11 sind über Abstandsrahmen 12, 13 an zwei rechtwinklig aneinander angrenzenden Seitenflächen eines, z. B. aus Aluminium bestehenden, quaderförmigen Metallblocks 14 montiert. Auf der von der Leuchtdiode 1 abgewandten Seitenfläche ist an dem Metallblock 14 eine Platte 15 mit dem hier ebenfalls verdeckten Referenzdetektor 4 montiert. Die Montage erfolgt über Schraubverbindungen, die durch gestrichelte Linien 16 angedeutet sind. Der Metallblock 14 wird von zwei Bohrungen 17, 18 durchsetzt, die zwischen den jeweils gegenüberliegenden Seitenflächen verlaufen. Auf seiner Deckfläche enthält der Metallblock 14 eine runde Öffnung 19, die in den Kreuzungsbereich der zwei Bohrungen 17, 18 hineinreicht. Der hier nicht sichtbare Strahlteiler 3 ist in einem im Wesentlichen zylinderförmigen Einsatz 20 angeordnet, der in die Öffnung 19 eingesetzt wird. Durch Drehen des Einsatzes 20 kann der Strahlteiler 3 in Bezug auf die Achsen der rechtwinklig verlaufenden Bohrungen 17, 18 justiert werden. Bei dem Stahlteiler 3 handelt es sich vorzugsweise um einen sogenannten Polka-Dot-Strahlteiler, bei dem eine transparente Trägerplatte im Flächenverhältnis von 50% mit metallischen Punkten bedampft ist.

Auf der von der Leuchtdiode 2 abgewandten Seitenfläche des Metallblocks 14 schließt sich an diesen die Messkammer 5 an, die als langgestreckter, hier quaderförmiger, beidseitig offener Hohlkörper 21 aus Metall, z. B. Aluminium, ausgebildet ist. Der Hohlkörper 21 selbst kann als Hohlprofilkörper ausgebildet sein oder eine spanend hergestellte durchgehende Bohrung 22 enthalten, die beidseitig mit Fenstern (hier ist nur das Fenster 23 sichtbar) abgeschlossen ist. Alternativ kann die Messkammer 5 mit teiltransparenten Hohlspiegeln abgeschlossen sein, die einen Resonator zur Verlängerung der Absorptionsstrecke bilden. Die Messkammer 5 weist auf der hier vom Betrachter abgewandten Seite zwei Zugangsöffnungen mit Anschlüssen für zwei Gasleitungen 24, 25 auf, über die das Messgas 7 zugeführt bzw. abgeführt wird. An der Außenwand der Messkammer 5 sind an gegenüberliegenden Stellen zwei Heizelemente angebracht, von denen hier nur ein Heizelement 26 in Form eines Transistors zu sehen ist, der zusammen mit dem anderen Heizelement von der Steuer- und Auswerteeinrichtung 8 zur Konstanthaltung der Temperatur der Messkammer 5 gesteuert bzw. geregelt wird.

Der Messdetektor 6 ist auf einer Platte 27 angeordnet und mit dieser an einer Rückseite eines metallenen Detektorblocks 28 montiert, der mit seiner Vorderseite an der von dem Metallblock 14 mit dem Strahlteiler 3 abgewandten Stirnseite der Messkammer 5 liegt. Zwischen der Vorder- und Rückseite des Detektorblocks 28 erstreckt sich eine Öffnung 29, die mit der Bohrung 22 der Messkammer 5 und der Bohrung 17 in dem Metallblock 14 fluchtet.

Auf den den Leuchtdioden 1, 2 zugewandten Seitenflächen des Metallblocks 14 sind in die Bohrungen 17, 18 Kollimatorlinsen 30, 31 mit den sie umgebenden Linsenhalterungen eingesetzt. Ebenso sind in der Bohrung 18 auf der dem Referenzdetektor 4 gegenüberliegenden Seitenfläche des Metallblocks 14 sowie auf der Rückseite des Detektorblocks 18 in der Öffnung 29 Fokussierlinsen 32, 33 mit den sie umgebenden Linsenhalterungen eingesetzt. Die Kollimatorlinsen 30, 31 formen das von den Leuchtdioden 1, 2 abgestrahlte Licht zu einem parallelen Lichtbündel, das mit Hilfe der Fokussierlinsen auf den Referenzdetektor 4 bzw. nach Durchstrahlen der Messkammer 5 auf den Messdetektor 6 fokussiert wird.

Der den Strahlteiler 3 enthaltende Metallblock 14, die Messkammer 5 und der Detektorblock 27 sind über vier Zugstangen 34 miteinander verspannt, die an ihren Enden Gewinde aufweisen und dort und an den nach außen gewandten Seiten der beiden Platten 11 und 27 mit diesen verschraubt sind. Im Bereich der Fenster 23 sind zwischen der Messkammer 5 und dem Metallblock 14 bzw. dem Detektorblock 28 Dichtringe 35 angeordnet. Die Zugstangen oder Zugstäbe 34 erstrecken sich in den Platten 11, 27, dem Abstandsrahmen 13, dem Metallblock 14 und dem Detektorblock 28 durch entsprechende Löcher oder Bohrungen 36, während sie entlang der Messkammer 5 in Nuten 37 verlaufen, die an der Außenwand des Hohlkörpers 21, hier an seinen Längskanten ausgebildet sind.

Die Steuer- und Auswerteeinrichtung 8 ist auf einer Platine 38 ausgebildet, die über der Messkammer 5 angeordnet ist.

Der Gasanalysator ist in einem Behälter aus Polystyrol-Hartschaum untergebracht, der aus einem schalenförmigen Unterteil 39 und einem schalen- oder deckelförmigen Oberteil 40 besteht.

Unter den die Leuchtdioden 1, 2 und Detektoren 4, 6 tragenden Platten 10, 11, 15, 27 sind nicht zwingenderweise Einzelplatten zu verstehen. Es kann sich auch jeweils um eine Kombination von z. B. zwei Einzelplatten handeln, wobei die eine Platte als Leiterplatte ausgebildet ist, die die Leuchtdiode bzw. den Detektor unmittelbar trägt und Anschlüsse zur elektrischen Verbindung mit der Steuer- und Auswerteeinrichtung 8 aufweist, während die andere Platte, z. B. aus Metall, die Leiterplatte aufnimmt und im Übrigen mechanisch stabil ausgebildet ist und zur Montage dient.

## Patentansprüche

1. Gasanalysator zur Messung von zwei Gaskomponenten in einem Messgas (7),
- mit einer ersten Leuchtdiode (1), die Licht mit einer ersten Wellenlänge im Bereich einer Absorptionslinie der einen, ersten Gaskomponente abstrahlt,
- mit einer zweiten Leuchtdiode (2), die Licht mit einer zweiten Wellenlänge im Bereich einer Absorptionslinie der anderen, zweiten Gaskomponente abstrahlt,
- mit einem Strahlteiler (3),
- mit einer von dem Messgas (7) durchströmbaren Messkammer (5), die als langgestreckter beidseitig offener Hohlkörper (21) aus Metall mit zwei seitlichen Zugangsöffnungen für das Messgas (7) ausgebildet ist,
- mit einem Messdetektor (6),
- mit einem Referenzdetektor (4) und
- mit einer Steuer- und Auswerteeinrichtung (8) zum abwechselnden Ansteuern der Leuchtdioden (1, 2) und zur Auswertung der von dem Messdetektor (6) und dem Referenzdetektor (4) gelieferten Signale zu Messergebnissen (9) für die beiden Gaskomponenten,
**dadurch gekennzeichnet, dass**
- der Gasanalysator ferner einen eine Grundfläche, eine Deckfläche und vier Seitenflächen aufweisenden quaderförmigen Metallblock (14) und einen eine Öffnung (29) enthaltenden Detektorblock (28) aus Metall umfasst, wobei
- der Strahlteiler (3) in einem Kreuzungsbereich von zwei Bohrungen (17, 18) des quaderförmigen Metallblocks (14) angeordnet ist, welche zwei Bohrungen (17, 18) zwischen den jeweils gegenüberliegenden Seitenflächen des quaderförmigen Metallblocks (18) verlaufen,
- die beiden Leuchtdioden (1, 2) an zwei aneinandergrenzenden Seitenflächen und die Messkammer (5) und der Referenzdetektor (4) an den beiden übrigen aneinandergrenzenden Seitenflächen des Metallblocks (14) jeweils gegenüber den Bohrungen (17, 18) angeordnet sind,
- der Messdetektor (6) an dem Detektorblock (28) gehalten ist und
- der quaderförmige Metallblock (14) und der Detektorblock (28) über Zugstangen (34) miteinander verbunden sind und die Messkammer (5) zwischen ihnen eingespannt ist.

2. Gasanalysator nach Anspruch 1, **dadurch gekennzeichnet, dass** die Zugstäbe (34) in Nuten (37) in der Außenwand des die Messkammer (5) bildenden Hohlkörpers (21) verlaufen.

3. Gasanalysator nach Anspruch 1 oder 2, **dadurch gekennzeichnet, dass** der die Messkammer (5) bildende Hohlkörper (21) als Hohlprofilkörper ausgebildet ist.

4. Gasanalysator nach einem der vorangehenden Ansprüche, **dadurch gekennzeichnet, dass** der quaderförmige Metallblock (14) auf seiner Deckfläche eine in den Kreuzungsbereich der zwei Bohrungen (17, 18) hineinreichende Öffnung (19) enthält und dass der Strahlteiler (3) in einem in die Öffnung (19) eingesetzten Einsatz (20) montiert ist.

5. Gasanalysator nach einem der vorangehenden Ansprüche, **dadurch gekennzeichnet, dass** die Leuchtdioden (1, 2) auf Platten (10, 11) gehalten sind, die unmittelbar oder unter Zwischenlage eines Abstandsrahmens (12, 13) an der jeweiligen Seitenfläche des quaderförmigen Metallblocks (14) montiert sind.

6. Gasanalysator nach einem der vorangehenden Ansprüche, **dadurch gekennzeichnet, dass** der Messdetektor (6) und Referenzdetektor (4) jeweils auf einer Platte (27, 15) gehalten sind, die unmittelbar oder unter Zwischenlage eines Abstandsrahmens an der jeweiligen Seitenfläche des quaderförmigen Metallblocks (14) bzw. der von der Messkammer (5) abgewandten Seite des Detektorblocks (21) montiert ist.

7. Gasanalysator nach einem der vorangehenden Ansprüche, **dadurch gekennzeichnet, dass** in den Bohrungen (17, 18) auf den den Leuchtdioden (1, 2) zugewandten Seitenflächen des Metallblocks (14) Kollimatorlinsen (30, 31) eingesetzt sind.

8. Gasanalysator nach einem der vorangehenden Ansprüche, **dadurch gekennzeichnet, dass** in der Bohrung (18) auf der dem Referenzdetektor (4) gegenüberliegenden Seitenfläche des Metallblocks (14) und in der Öffnung (29) des Detektorblocks (28) jeweils eine Fokussierlinse (32, 33) eingesetzt ist.

9. Gasanalysator nach einem der vorangehenden Ansprüche, **dadurch gekennzeichnet, dass** an der Außenwand des die Messkammer (5) bildenden Hohlkörpers (21) mindestens ein elektrisches Heizelement (26) montiert ist.

10. Gasanalysator nach einem der vorangehenden Ansprüche, **dadurch gekennzeichnet, dass** er in einem aus einem schalenförmigen Unterteil (39) und einem schalen- oder deckelförmigen Oberteil (40) bestehenden Behälter aus Dämmstoff angeordnet ist.

11. Gasanalysator nach einem der vorangehenden Ansprüche, **dadurch gekennzeichnet, dass** die Messkammer (5) mit teiltransparenten Hohlspiegeln abgeschlossen ist, die zwischen sich einen Resonator zur Verlängerung der Absorptionsstrecke bilden.

## Claims

1. Gas analyser for measuring two gas components in a measurement gas (7),
- with a first light-emitting diode (1) which emits light with a first wavelength in the range of an absorption line of the one, first gas component,
- with a second light-emitting diode (2) which emits light with a second wavelength in the range of an absorption line of the other, second gas component,
- with a beam splitter (3),
- with a measurement chamber (5), through which measurement gas (7) can flow, said measurement chamber (5) being embodied as an elongated hollow body (21) open on both sides and consisting of metal, with two lateral access openings for the measurement gas (7),
- with a measurement detector (6),
- with a reference detector (4) and
- with a control and evaluation device (8) for controlling the light-emitting diodes (1, 2) in an alternating manner and for evaluating the signals supplied by the measurement detector (6) and the reference detector (4) to form measurement results (9) for the two gas components,
**characterised in that**
- the gas analyser further comprises a rectangular metal block (14) comprising a base, a top surface and four lateral surfaces and a detector block (28) which is made from metal and contains an opening (29), wherein
- the beam splitter (3) is arranged in an intersection region of two bores (17, 18) of the rectangular metal block (14), which two bores (17, 18) run between the respective opposing lateral surfaces of the rectangular metal block (18),
- the two light-emitting diodes (1, 2) are arranged on two adjoining lateral surfaces and the measurement chamber (5) and the reference detector (4) are arranged on the other two adjoining lateral surfaces of the metal block (14) in each case opposing the bores (17, 18),
- the measurement detector (6) is held on the detector block (28) and
- the rectangular metal block (14) and the detector block (28) are connected to one another via tension rods (34) and the measurement chamber (5) is clamped between them.

2. Gas analyser according to claim 1, **characterised in that** the tension rods (34) run in grooves (37) in the external wall of the hollow body (21) forming the measurement chamber (5).

3. Gas analyser according to claim 1 or 2, **characterised in that** the hollow body (21) forming the measurement chamber (5) is embodied as a hollow profile body.

4. Gas analyser according to one of the preceding claims, **characterised in that** the rectangular metal block (14) contains on its top surface an opening (19) extending into the intersection region of the two bores (17, 18) and **in that** the beam splitter (3) is mounted in an insert (20) inserted in the opening (19).

5. Gas analyser according to one of the preceding claims, **characterised in that** the light-emitting diodes (1, 2) are held on plates (10, 11) which are mounted directly or with the intermediate layering of a spacer frame (12, 13) on the respective lateral surface of the rectangular metal block (14) .

6. Gas analyser according to one of the preceding claims, **characterised in that** the measurement detector (6) and reference detector (4) are each held on a plate (27, 15) which is mounted directly or with the intermediate layering of a spacer frame on the respective lateral surface of the rectangular metal block (14) or the side of the detector block (21) facing away from the measurement chamber (5).

7. Gas analyser according to one of the preceding claims, **characterised in that** collimator lenses (30, 31) are inserted in the bores (17, 18) on the lateral surfaces of the metal block (14) facing the light-emitting diodes (1, 2).

8. Gas analyser according to one of the preceding claims, **characterised in that** a focusing lens (32, 33) is inserted in each case in the bore (18) on the lateral surface of the metal block (14) opposing the reference detector (4) and in the opening (29) of the detector block (28).

9. Gas analyser according to one of the preceding claims, **characterised in that** at least one electric heating element (26) is mounted on the external wall of the hollow body (21) forming the measurement chamber (5).

10. Gas analyser according to one of the preceding claims, **characterised in that** it is arranged in a container made of insulating material consisting of a shell-shaped lower part (39) and a shell-shaped or cover-shaped upper part (40).

11. Gas analyser according to one of the preceding claims, **characterised in that** the measurement chamber (5) is closed with semitransparent concave mirrors which between them form a resonator to extend the absorption path.

## Revendications

1. Analyseur de gaz pour la mesure de deux constituants gazeux d'un gaz (7) à mesurer,
- comprenant une première diode (1) électroluminescente, qui rayonne de la lumière ayant une première longueur d'onde dans le domaine d'un raie d'absorption d'un premier constituant gazeux,
- comprenant une deuxième diode (2) électroluminescente, qui rayonne de la lumière ayant une deuxième longueur d'onde dans le domaine d'un raie d'absorption de l'autre deuxième constituant gazeux,
- comprenant un diviseur (3) de faisceau,
- comprenant une chambre (5) de mesure, dans laquelle le gaz (7) à mesurer peut passer, et qui est constituée sous la forme d'un corps (21) creux s'étendant en longueur, ouvert des deux côtés, en métal, ayant deux ouvertures latérales d'accès du gaz (7) à mesurer,
- comprenant un détecteur (6) de mesure,
- comprenant un détecteur (4) de référence et
- comprenant un dispositif (8) de commande et d'analyse pour la commande alternée des diodes (1, 2) électroluminescentes et pour la transformation des signaux fournis par le détecteur (6) de mesure et le détecteur (4) de référence en des résultats (9) de mesure pour les deux constituants gazeux,
**caractérisé en ce que**
- l'analyseur de gaz comprend en outre un bloc (14) métallique parallélépipédique comportant une surface de base, une surface supérieure et quatre surfaces latérales et un bloc (28) de détecteur en métal comportant une ouverture (29), dans lequel
- le diviseur (3) de faisceau est monté dans une partie de croisement de deux trous (17, 18) du bloc (14) métallique parallélépipédique, lesquels deux trous (17, 18) s'étendent entre les surfaces latérales opposées respectivement du bloc (18) métallique parallélépipédique,
- les deux diodes (1, 2) électroluminescentes sont disposées sur deux surfaces latérales voisines l'une de l'autre et la chambre (5) de mesure et le détecteur (4) de référence sur les deux autres surfaces latérales voisines l'une de l'autre du bloc (14) métallique respectivement en regard des trous (17, 18),
- le détecteur (6) de mesure est retenu sur le bloc (28) de détecteur, et
- le bloc (14) métallique parallélépipédique et le bloc (28) de détecteur sont reliés entre eux par des tirants (34) et la chambre (5) de mesure est serrée entre eux.

2. Analyseur de gaz suivant la revendication 1, **caractérisé en ce que** les tirants (34) s'étendent dans des rainures (37) de la paroi extérieure du corps (21) creux formant la chambre (5) de mesure.

3. Analyseur de gaz suivant la revendication 1 ou 2, **caractérisé en ce que** le corps (21) creux formant la chambre (5) de mesure est constitué sous la forme d'un corps profilé creux.

4. Analyseur de gaz suivant l'une des revendications précédentes, **caractérisé en ce que** le bloc (14) métallique parallélépipédique comporte sur sa surface supérieure une ouverture (19) allant jusqu'à la partie de croisement des deux trous (17, 18), et **en ce que** le diviseur (3) de faisceau est monté dans un insert (20) inséré dans l'ouverture (19).

5. Analyseur de gaz suivant l'une des revendications précédentes, **caractérisé en ce que** les diodes (1, 2) électroluminescentes sont retenues sur des plaques (10, 11), qui sont montées directement ou avec interposition d'un cadre (12, 13) intercalaire sur les surfaces latérales respectives du bloc (14) métallique parallélépipédique.

6. Analyseur de gaz suivant l'une des revendications précédentes, **caractérisé en ce que** le détecteur (6) de mesure et le détecteur (4) de référence sont retenus respectivement sur une plaque (27, 15), qui est montée directement ou avec interposition d'un cadre intercalaire sur les surfaces latérales respectives du bloc (14) métallique parallélépipédique ou sur le côté, non tourné vers la chambre (5) de mesure, du bloc (21) de détecteur.

7. Analyseur de gaz suivant l'une des revendications précédentes, **caractérisé en ce que** des lentilles (30, 31) collimatrices sont insérées dans les trous (17, 18) sur les surfaces latérales, tournées vers les diodes (1, 2) électroluminescentes, du bloc (14) métallique.

8. Analyseur de gaz suivant l'une des revendications précédentes, **caractérisé en ce que** respectivement une lentille (32, 33) de focalisation est insérée dans le trou (18), sur la surface latérale en face du détecteur (4) de référence du bloc (14) métallique, et dans l'ouverture (29) du bloc (28) de détecteur.

9. Analyseur de gaz suivant l'une des revendications précédentes, **caractérisé en ce qu'**au moins un élément (26) de chauffage électrique est monté sur la paroi extérieure du corps (21) creux formant la chambre (5) de mesure.

10. Analyseur de gaz suivant l'une des revendications précédentes, **caractérisé en ce qu'**il est disposé dans un récipient en une matière calorifuge constitué d'une partie (39) inférieure en forme de cuvette et d'une partie (40) supérieure en forme de cuvette ou de couvercle.

11. Analyseur de gaz suivant l'une des revendications précédentes, **caractérisé en ce que** la chambre (5) de mesure est fermée par des miroirs concaves semi-transparents, qui forment entre eux un résonnateur de rallongement de la région d'absorption.
